## Europäisches Patentamt
## European Patent Office
## Office européen des brevets

(11) Numéro de publication: **0 112 754**
**B1**

(12) **FASCICULE DE BREVET EUROPÉEN**

(45) Date de publication du fascicule du brevet:
**05.11.86**

(51) Int. Cl.⁴: **B 01 J 23/86,** C 07 C 5/32, C 07 C 15/46

(21) Numéro de dépôt: **83402346.7**

(22) Date de dépôt: **06.12.83**

(54) Catalyseur contenant des oxydes de fer, de chrome, de potassium et de lanthane, sa préparation et son utilisation dans des réactions de déshydrogénation.

(30) Priorité: **14.12.82 FR 8221086**
**14.12.82 FR 8221085**

(43) Date de publication de la demande:
**04.07.84 Bulletin 84/27**

(45) Mention de la délivrance du brevet:
**05.11.86 Bulletin 86/45**

(84) Etats contractants désignés:
**DE IT NL**

(56) Documents cité:
**FR-A-2 387 200**
**US-A-3 595 809**
**US-A-4 134 858**

(73) Titulaire: **INSTITUT FRANCAIS DU PETROLE, 4, Avenue de Bois- Préau, F-92502 Rueil- Malmaison (FR)**

(72) Inventeur: **Courty, Philippe, 91, rue Condorcet, F-78800 Houilles (FR)**
Inventeur: **Roussel, Michel, 45, avenue Guillebaud, F-92160 Antony (FR)**
Inventeur: **Varin, Philippe, 6, rue de la Saussaye, F-91300 Massy (FR)**
Inventeur: **Le Page, Jean- François, 13, rue des Primevères, F-92500 Rueil- Malmaison (FR)**
Inventeur: **Leporq, Serge, 6, rue Villandry, F-78200 Mantes la Ville (FR)**

LIBER, STOCKHOLM 1986

## Description

La présente invention concerne de nouveaux catalyseurs utilisables dans des réactions de déshydrogénation et en particulier dans la déshydrogénation des hydrocarbures aliphatiques saturés ou monoéthyléniques à faible poids moléculaire (contenant par exemple de 2 à 8 atomes de carbone), ainsi que dans la déshydrogénation des hydrocarbures alkylaromatiques (tels que l'éthylbenzène ou les diéthylbenzènes), en hydrocarbures aromatiques vinyliques (tels que le styrène ou le divinylbenzène). Elle concerne également la préparation de ces catalyseurs, ainsi que des procédés de déshydrogénation dans lesquels on les utilise.

Il est connu que, dans la déshydrogénation des hydrocarbures précités, on fait passer l'hydrocarbure, de préférence additionné d'une forte proportion de vapeur d'eau (1 à 30 moles $H_2O$/mole hydrocarbure), sur un catalyseur à une vitesse volumétrique horaire, rapportée à l'hydrocarbure liquide, de 0,05 à 5, de préférence de 0,1 à 1 volume (TPN), par volume de catalyseur et par heure et à une température d'environ 450 à 750°C.

On a déjà décrit dans l'art antérieur des catalyseurs pour la déshydrogénation d'hydrocarbures oléfiniques, tels que les butènes (en butadiène) ou d'hydrocarbures alkylaromatiques tels que l'éthyl benzène (en styrène), ces catalyseurs renfermant d'une proportion pondérale majeure d'oxyde de fer, un composé (oxyde ou carbonate) de potassium, de l'oxyde de vanadium, éventuellement de l'oxyde de chrome, ainsi qu'une faible proportion (de 0,01 à 10 % en poids) d'au moins un oxyde additionnel d'un métal tel que l'aluminium, le cadmium, le cuivre, le magnésium, le manganèse, le nickel, les métaux des terres rares, l'uranium et le zinc. De tels catalyseurs sont décrits notamment dans FR-A-2 387 200 qui correspond en substance aux brevets des Etats-Unis 4,143,083 et 4,152,300. Dans ces brevets, les métaux des terres rares sont définis comme étant les métaux de numéros atomiques de 58 à 71 inclus, c'est-à-dire du cérium au lutétium. Dans les exemples, les métaux des terres rares utilisés sont le cérium, le praséodyme et le néodyme.

Or, on a maintenant découvert qu'il était avantageux d'utiliser comme métal de terre rare dans des catalyseurs de ce type le lanthane (de numéro atomique 57), celui-ci apportant une amélioration des propriétés catalytiques vis-à-vis des réactions de déshydrogénation (notamment de l'éthylbenzène en styrène) pour un coût plus réduit.

Il a également été décrit, dans FR-A-2 270 003, auquel correspond en substance le brevet des Etats-Unis 4,134,858, la mise en jeu d'un matériau argileux dans la fabrication d'un catalyseur de déshydrogénation principalement à base d'oxydes de fer, de chrome et de potassium. Lors de l'activation thermique, à une température convenable, généralement comprise entre 850 et 1100°C, le matériau argileux se combine à l'oxyde de potassium pour former un silicate double d'aluminium et de potassium (plus particulièrement de la kaliophyllite). Cette technique permet notamment de diminuer la densité de remplissage du catalyseur.

L'objet principal de la présente invention est de décrire des catalyseurs améliorés qui présentent une activité et/ou une sélectivité accrues vis-à-vis des catalyseurs décrits dans l'art antérieur. Leur durée de vie est sensiblement égale, souvent supérieure, à celle des catalyseurs connus.

Un autre objet de l'invention est de décrire un procédé de préparation desdits catalyseurs.

Un autre objet de l'invention est de décrire l'emploi de ces catalyseurs dans les réactions de déshydrogénation d'hydrocarbures.

Ces objets sont atteints par les catalyseurs de l'invention définis d'une manière générale comme renfermant des oxydes de fer, de chrome et de potassium présents dans des rapports pondéraux:

$$\frac{Fe_2O_3}{K_2O} \quad : \text{de } 1/1 \text{ à } 10/1, \text{ de préférence de } 2/1 \text{ à } 7/1 \ ;$$

$$\frac{CrO_3}{K_2O} \quad : \text{de } 0,05/1 \text{ à } 0,4/1, \text{ de préférence de } 0,1/1 \text{ à } 0,3/1 ; \text{et}$$

$$\frac{Fe_2O_3}{CrO_3} \quad : \text{ de } 15/1 \text{ à } 40/1, \text{ de préférence de } 25/1 \text{ à } 35/1 \text{ ;}$$

éventuellement un silicate double d'aluminium et de potassium, dont la composition est plus particulièrement $Al_2O_3$, $2\,SiO_2$, $K_2O$ (kaliophyllite), en une proportion de 5 à 40 % en poids;
et une proportion d'oxyde de lanthane de 1 à 15 % en poids, de préférence de 3 à 10 % en poids.

Lorsque le catalyseur renferme un silicate double d'aluminium et de potassium, il contient une quantité d'oxyde de potassium en excès par rapport à la quantité combinée sous forme de silicate double.

Dans ce cas, l'excès de potassium, compté en oxyde, est avec l'oxyde de fer et l'oxyde de chrome dans des rapports pondéraux suivants:

$$\frac{Fe_2O_3}{\text{excès de } K_2O} \quad : \text{ de } 3/1 \text{ à } 10/1 \text{ ; et}$$

$$\frac{CrO_3}{\text{excès de } K_2O} \quad : \text{ de } 0,1/1 \text{ à } 0,4/1.$$

Les catalyseurs de l'invention peuvent en outre contenir une faible proportion, par exemple de 0,1 à 5% en poids, d'un oxyde de métal alcalin autre que le potassium ou d'un métal alcalino-terreux (par exemple $Na_2O$, BaO, CaO).

Le catalyseur peut également contenir, en une proportion en oxyde de 0,1 à 5% de son poids, au moins un métal tel que le cobalt ou le vanadium.

Il est en outre avantageux que le lanthane soit présent dans le catalyseur au moins en partie sous une forme combinée en oxyde mixte avec au moins un métal tel que le cobalt ou le vanadium. L'oxyde mixte est plus particulièrement du type pérovskite.

Pour préparer les catalyseurs de l'invention, on forme un mélange comprenant de l'eau, au moins un composé du fer, au moins un composé du chrome, au moins un composé du potassium et au moins un composé de lanthane, dans des proportions qui correspondent, en oxydes, aux rapports pondéraux suivants:

$$\frac{Fe_2O_3}{K_2O} \quad : \text{ de } 1/1 \text{ à } 10/1, \text{ de préférence de } 2/1 \text{ à } 7/1,$$

$$\frac{CrO_3}{K_2O} \quad : \text{ de } 0,05/1 \text{ à } 0,4/1, \text{ de préférence de } 0,1/1 \text{ à } 0,3/1,$$

$$\frac{Fe_2O_3}{CrO_3} \quad : \text{de } 15/1 \text{ à } 40/1, \text{ de préférence de } 25/1 \text{ à } 35/1,$$

Le composé de lanthane représentant, compté en oxyde, de 1 à 15 %, de préférence de 3 à 10 % en poids par rapport à l'ensemble des réactifs comptés en oxydes.

Lorsque l'on met en jeu un matériau argileux, celui-ci représente en poids une proportion d'environ 3,5 à 30 % par rapport à l'ensemble des ingrédients mis en jeu, comptés en oxydes; le composé du potassium étant mis en jeu en excès par rapport à la quantité susceptible de se combiner audit matériau argileux, l'excès de potassium étant avec le fer et le chrome, comptés en oxydes, dans les rapports pondéraux suivants:

$$\frac{Fe_2O_3}{\text{excès de } K_2O} \quad : \text{ de } 3/1 \text{ à } 10/1 \text{ ; et}$$

$$\frac{CrO_3}{\text{excès de } K_2O} \quad : \text{ de } 0,1/1 \text{ à } 0,4/1 \text{ ;}$$

On malaxe ensuite le mélange précédent de manière à obtenir une pâte. Le malaxage peut se faire au moyen d'un malaxeur HOBBART ou d'un appareil WARRING-BLENDER.

La pâte obtenue peut alors être mise en forme, par exemple par extrusion, par exemple dans une extrudeuse ANDOUART, HUTTE, ALEXANDER WERKE, O'TOOLE ou WERNER PFLEI-DERER. Les extrudés peuvent consister par exemple en des cylindres de diamètre et de hauteur compris entre 1 et 7 mm. Le séchage est en général effectué à l'étuve et l'activation thermique peut être menée par exemple dans un four électrique ou dans un four à gaz de combustion. Le séchage peut être effectué par exemple à 100-300°C pendant 1 à 24 heures, et la calcination effectuée à 850-1.100°C, de préférence 870 à 1.050°C, pendant 1 à 3 heures.

Le composé du fer utilisé est en général de l'oxyde de fer ferrique, finement broyé (par exemple en grains de taille inférieure à 200 mesh). Cet oxyde de fer est avantageusement précalciné à 800°C. On utilise par exemple les produits commercialisés par la Société Française d'Electrométallurgie (SOFREM) sous la référence "HA 160". Cependant, il est possible de remplacer une partie, par exemple de 5 à 20% en poids de l'oxyde de fer total par un hydroxyde de fer, obtenu lui-même par précipitation ammoniacale, sodique ou potassique d'une solution aqueuse d'un sel de fer. On peut également remplacer une partie, par exemple de 5 à 20% en poids de l'oxyde de fer total par un sel de fer thermiquement décomposable tel qu'un sulfate, un carbonate, un acétate, un oxalate, un citrate, un nitrate ou un alun de fer. On peut alors utiliser les sels de fer ferreux ou les sels de fer ferrique.

Les composés du chrome, utilisés seuls ou en mélange, sont en général l'anhydride chromique, les sels chromiques, par exemple chromate ou bichromate de sodium, de potassium ou d'ammonium, les composés du chrome $+3$, par exemple nitrate, sulfate, oxyde, ou hydroxyde.

Les composés utilisés pour introduire le potassium, les autres métaux alcalins ou alcalino-terreux éventuels (Na, Ca, Ba) ainsi que le cobalt et le vanadium sont en général les carbonates, les oxydes et les hydroxydes. On peut encore utiliser d'autres sels, par exemple les sulfates, les phosphates ou les nitrates. Pour le vanadium, on peut encore utiliser les vanadates ou métavanadates.

Le lanthane peut être introduit sous forme d'oxyde ou d'hydroxyde, ou sous forme d'um composé susceptible de se convertir en oxyde par chauffage à une température inférieure ou égale à celle choisie pour le chauffage du catalyseur, lors de sa préparation. Des exemples de tels composés sont les carbonates, nitrates, nitrites, sulfures, et les sels d'acides organiques, par exemple les formiates, acétates, oxalates, tartrates, citrates, acétylacétonates, benzoates desdits éléments.

Des exemples de matériaux argileux utilisés éventuellement pour préparer les catalyseurs de l'invention ont été donnés dans FR-A-2 270 003 cité plus haut. On utilisera de préférence la kaolinite, par exemple sous la forme de kaolin de Cornouailles.

4

Enfin, on pourra mettre en jeu une proportion mineure d'un agent favorisant l'extrusion. De tels matériaux sont connus de l'homme de l'art. On peut citer par exemple: le graphite, des gommes végétales (gomme arabique, gomme Damhar, gomme de Caroube) ou des alkylcelluloses (méthyl-éthyl- ou carboxyméthylcelluloses).

Tous les ingrédients individuels du catalyseur peuvent être mélangés à l'eau pour former une pâte qui est ensuite séchée ou calcinée, ou bien certains ingrédients peuvent être introduits après avoir subi um traitement préalable destiné à former des combinaisons chimiques entre eux.

Ainsi, le lanthane peut être introduit sous la forme du produit résultant de la réaction préalable d'au moins un composé de lanthane avec au moins un composé un métal (M), en particulier le cobalt ou le vanadium.

Dans cette étape préalable, le composé de lanthane et le(s) composé(s) de métal M sont en général mis en jeu dans des proportions correspondant à un rapport atomique La/M de 0,5/1 à 2/1, et plus particulièrement d'environ 1/1.

Dans le cas du cobalt, on peut effectuer une réaction en solution entre sels solubles conduisant à un précipité, cette réaction étant suivie d'une séparation du produit obtenu (par exemple par filtration et lavage du précipité), d'un séchage et d'un chauffage par exemple à une température de 400 à 950°C. On forme alors au moins en partie un oxyde mixte La Co O$_3$ de type perovskite.

On peut encore opérer par réaction entre oxydes ou entre espèces thermiquememt décomposables en oxydes du lanthane d'une part et du cobalt ou du vanadium d'autre part. Dans le cas du vanadium, mis en jeu sous la forme d'un composé de vanadium 5+, on opère sous atmosphère réductrice (hydrogène) à température élevée, par exemple de 600 à 900° C. Dans tous les cas, on procède ensuite à une calcination à une température de 900 à 1.250°C. Le produit obtenu est au moins en partie sous la forme d'un oxyde mixte de type perovskite.

Le produit obtenu dans cette étape préalable est ensuite mélangé aux autres ingrédients et à l'eau et la préparation du catalyseur est alors poursuivie de la manière indiquée plus haut, par séchage et chauffage à température élevée (par exemple de 850 à 1.100°C).

Les catalyseurs de l'invention, tels que définis ci-dessus, sont utilisés dans la déshydrogénatiom d'hydrocarbures, en particulier d'hydrocarbures aliphatiques saturés ou monoéthyléniques, et d'hydrocarbures alkylaromatiques (éthylbenzène, diéthylbenzènes), dans des conditions opératoires connues en soi et rappelées plus haut.

Dans la déshydrogénation de l'éthylbenzène en styrène, notamment, les catalyseurs de l'invention permettent d'obtenir une meilleure conversion de l'éthylbenzène et/ou une meilleure sélectivité en styrène.

Les exemples suivants illustrent l'invention Ils ne doivent en aucune manière être considérés comme limitatifs. Les exemples 3 à 8, 11, 12, et 15 sont donnés à titre de comparaison.

## Exemple 1

(Catalyseur A)
On malaxe dans un appareil WARRING-BLENDER, 300 g d'oxyde de fer ferrique "HA 160", 14,5 g de bichromate de potassium, 100 g de carbonate de potassium anhydre et 27 g d'oxyde de lanthane La$_2$O$_3$.
Les quantités de réactifs utilisées correspondent, en oxydes, aux proportions suivantes (% poids):
Fe$_2$O$_3$ = 73,23%
CrO$_3$ = 2,41%
K$_2$O = 17,77%
La$_2$O$_3$ = 6,59%
Les rapports pondéraux entre le fer, le chrome et le potassium, comptés en oxydes, sont les suivants:

$$\frac{Fe_2O_3}{K_2O} = 4,12 \quad ; \quad \frac{CrO_3}{K_2O} = 0,135 \quad ; \quad \frac{Fe_2O_3}{CrO_3} = 30,4$$

On ajoute ensuite 67 ml d'une solution aqueuse à 2% en poids de Méthocel (Commercialisé par DOW CHEMICALS) et 70 ml d'eau, de façon à obtenir une pâte consistante, qui est malaxée durant 20 minutes puis transférée dans une extrudeuse à piston et extrudée en joncs de 4 mm de diamètre et de 2 à 6 mm de longueur.

Les extrudés sont séchés à 250°C pendant 15 heures puis activés pendant 2 heures a 960°C. On obtient ainsi le catalyseur A.

L'analyse du catalyseur fini confirme sa teneur en oxydes de fer, de chrome, de potassium et de lanthane.

Dans tous les exemples qui suivent, le malaxage des ingrédients, l'extrusion et le séchage des catalyseurs sont effectués dans les mêmes conditions que dans l'exemple 1. L'activation des catalyseurs est effectuée par chauffage pendant 2 heures à des températures qui s'échelonnent de 940 à 970°C.

**Exemple 2**

(Catalyseur B).

On malaxe dans un appareil WARRING-BLENDER, 300 g d'oxyde de fer ferrique "HA 160", 14,5 g de bichromate de potassium, 100 g de carbonate de potassium anhydre, 28,5 g de Kaolin de Cornouailles et 27 g d'oxyde de lanthane $La_2O_3$. Les quantités de réactifs utilisées correspondent, en oxydes, aux pourcentages pondéraux suivants:

$Fe_2O_3 = 68,47\%$
$CrO_3 = 2,25\%$
$K_2O = 16,62\%$
$La_2O_3 = 6,16\%$
Kaolin $= 6,50\%$

Les rapports pondéraux entre le fer, le chrome et le potassium comptés en oxydes, ont des valeurs voisines de celles indiquées dans l'exemple 1.

La quantité de potassium susceptible de se combiner avec le kaolin correspond à une proportion pondérale, en oxyde, de 2,76% par rapport à l'ensemble des oxydes. La proportion d'oxyde de potassium "en excès" est donc de 13,86%.

Les rapports entre l'oxyde de fer et l'oxyde de chrome d'une part et l'excès de $K_2O$ d'autre part sont respectivement les suivants: (rapports pondéraux):

$$\frac{Fe_2O_3}{\text{excès de } K_2O} = 4,94 \quad ; \quad \frac{CrO_3}{\text{excès de } K_2O} = 0,162$$

On ajoute ensuite 67 ml d'une solution aqueuse à 2% en poids de Méthocel (commercialisé par DOW CHEMICALS) et 70 ml d'eau, de façon à obtenir une pàte consistante.

On procéde ensuite comme indiqué dans l'exemple 1.

L'analyse du catalyseur fini confirme sa teneur en oxydes de fer, de chrome, de potassium et de lanthane. L'analyse par diffraction des Rayons X permet de mettre en évidence la présence de silicate double d'aluminium et de potassium (kaliophyllite) dont la teneur pondérale est de 9,3%.

**Exemples 3 à 5**

(Catalyseurs C à E).

On répète la préparation décrite dans l'exemple 1, à la différence près que l'on utilise, au lieu de l'oxyde de lanthane $La_2O_3$, une même quantité pondérale d'un autre oxyde de terre rare:

**exemple 3**

: l'oxyde de cérium $CeO_2$ (catalyseur C).

**exemple 4**

: l'oxyde de praséodyme $Pr_8O_{11}$ (catalyseur D),

**exemple 5**

: l'oxyde de néodyme $NdO_3$ (catalyseur E).

6

**Exemples 6 à 8**

(Catalyseurs F à H)

On répète la préparation décrite dans l'exemple 2 (catalyseur B), à la différence près que l'on utilise, au lieu de l'oxyde de lanthane $La_2O_3$, une même quantité pondérale d'un autre oxyde de terre rare:

**Exemple 6**

: L'oxyde de cérium $CeO_2$ (catalyseur F)

**Exemple 7**

: l'oxyde de praséodyme $Pr_8O_{11}$ (catalyseur G)

**Exemple 8**

: l'oxyde de néodyme $NdO_3$ (catalyseur H)

**Exemple 9**

(Catalyseur I)

On malaxe dans un appareil WARRING-BLENDER, 300 g d'oxyde de fer ferrique "HA 160", 14,5 g de bichromate de potassium 100 g de carbonate de potassium anhydre, 28,5 g de Kaolin de Cornouailles, 17,9 g d'oxyde de lanthane $La_2O_3$ 9,1 g d'oxyde de cobalt $Co_2O_3$. On a choisi les mêmes quantités atomiques de lanthane et de cobalt.

Les quantités de réactifs mises en jeu correspondent, en oxydes, aux pourcentages suivants (% poids):

$Fe_2O_3$ = 68,47%
$CrO_3$ = 2,25%
$K_2O$ = 16,62%
$La_2O_3$ = 4,08%
$Co_2O_3$ = 2,08%
Kaolin = 6,50%

On ajoute 50 ml d'une solution aqueuse à 2% en poids de Méthocel et 100 ml d'eau de façon à obtenir une pâte qui est malaxée durant 15 minutes. L'extrusion, le séchage et la calcination du catalyseur sont effectués comme indiqué précédemment.

La teneur en silicate double d'aluminium et de potassium (kaliophyllite) dans les catalyseurs finis est voisine de 9,3% en poids.

**Exemple 10**

(Catalyseur J)

On répète la préparation décrite dans l'exemple 8 (catalyseur I) à la différence près que l'on utilise au lieu de l'oxyde de cobalt $Co_2O_3$, une même quantité pondérale d'anhydride vanadique $V_2O_5$.

Les quantités de réactifs mis en jeu correspondent, en oxydes, aux proportions suivantes (% poids):

$Fe_2O_3$ = 68,47 %
$CrO_3$ = 2,25 %
$K_2O$ = 16,62 %
$La_2O_3$ = 4,08 %
$V_2O_5$ = 2,08 %
Kaolin = 6,50 %.

Les rapports pondéraux entre le fer, le chrome et le potassium, comptés en oxydes, ont des valeurs voisines de celles indiquées dans l'exemple 1.

L'analyse du catalyseur fini confirme les teneurs en oxydes de fer, de chrome, de potassium, de lanthane et de vanadium.

**Exemple 11**

(Catalyseur K)

On répète la préparation décrite dans l'exemple 9, (catalyseur I) à la différence près que l'on utilise, au lieu de l'oxyde de lanthane, une même quantité pondérale d'oxyde de cérium $CeO_2$.

**Exemple 12**

(Catalyseur L)

On répète la préparation décrite dans l'exemple 10 (catalyseur J) à la différence près que l'on utilise, au lieu de l'oxyde de lanthane $La_2O_3$, une même quantité pondérale d'oxyde de cérium $CeO_2$.

**Exemple 13**

(Catalyseur M)

145,5 g de nitrate de cobalt et 190 ml d'une solution aqueuse à 2,63 mole/l de nitrate de lanthane sont versés dans 2,5 l d'eau à 80°C renfermant 212 g de $Na_2CO_3$.

Les quantités de réactifs mises en jeu correspondent à un rapport atomique du cobalt au lanthane de 1/1.

On obtient un précipité, qu'on lavé par 12 litres d'eau distillée. On laisse mûrir durant 6 heures à 80°C, on filtre et on sèche 24 heures à 50°C, 72 heures à 100°C puis 24 heures à 120°C. Enfin, on chauffe pendant 2 heures à 600°C.

La structure du produit obtenu n'est pas identifiée avec certitude. Il semble qu'il s'agit, au moins partiellement, d'un oxyde mixte du type pérovskite $LaCoO_3$.

On mélange 27 g du produit ainsi préparé avec 300 g d'oxyde de fer ferrique "HA 160", 14,5 g de bichromate de potassium, 100 g de carbonate de potassium anhydre et 28,5 g de kaolin de Cornouailles.

Les quantités de réactifs mises en jeu correspondent, en oxydes, aux pourcentages suivants (% poids):

$Fe_2O_3 = 68,47\%$
$CrO_3 = 2,25\%$
$K_2O = 16,62\%$
$LaCoO_3 = 6,16\%$
Kaolin $= 6,50\%$

On procède ensuite dans les mêmes conditions qu'indiquées précédemment.

**Exemple 14**

(catalyseur N)

163 g d'oxyde de lanthane $La_2O_3$ et 91 g d'anhydride vanadique $V_2O_5$ sont mélangés intimement puis chauffés dans un four tubulaire sous courant d'hydrogène â 850°C pendant 6 heures et enfin calcinés 18 heures à 1.200°C. Les quantités des réactifs mises en jeu correspondent à un rapport atomique La/V d'environ 1/1. Le produit obtenu consiste au moins en partie en un oxyde mixte de type perovskite $LaVO_3$.

On opère ensuite comme décrit dans l'exemple 13.

**Exemple 15**

(catalyseur O)

On répète la préparation décrite dans l'exemple 14 (catalyseur N), à la différence près que l'on utilise, au lieu d'oxyde mixte formé préalablement entre le lanthane et le vanadium, une même quantité pondérale d'un oxyde mixte formé préalablement entre le cérium et le vanadium, dans des proportions atomiques Ce/V d'environ 1/1.

Les catalyseurs A à O ont fait l'objet d'un test catalytique de longue durée.

Le test catalytique est effectué dans un "catatest" qui fonctionne sous pression atmosphérique et est alimenté en éthylbenzène industriel et en eau. Le volume de catalyseur testé est 100 ml (60 à 120 g cata). Les catalyseurs A à 0 sont sous la forme d'extrudés de 4 mm de diamètre et de 4-5mm de longueur.

Le catalyseur est tout d'abord préchauffé jusqu'à environ 500°C. On introduit alors la vapeur d'eau puis, vers environ 550°C, on introduit l'éthylbenzène. Les températures sont alors ajustées pour avoir une température de $614 \pm 2°C$ dans le lit catalytique.

Les débits horaires choisis sont les suivants:

$$\frac{\text{Ethylbenzène}}{\text{catalyseur}} = 0,4 \text{ volume } v^{-1} h^{-1} \qquad \frac{H_2O}{\text{Ethylbenzène}} = 2g \ g^{-1}$$

On indique au tableau 1 la conversion de l'éthylbenzène ($C_{EB}$), la sélectivité en styrène ($S_{ST}$) et le rendement en styrène ($R_{ST}$). Ces différents chiffres sont exprimés en % molaire et correspondent aux définitions suivantes:

$$C_{EB} = \frac{\text{moles d'éthylbenzène transformées}}{\text{moles d'éthylbenzène engagées}} \times 100$$

$$S_{ST} = \frac{\text{moles d'éthylbenzène transformées en styrène}}{\text{moles d'éthylbenzène transformées}} \times 100$$

$$R_{ST} = \frac{\text{moles de styrène produites}}{\text{moles d'éthylbenzène engagées}} \times 100$$

Ces grandeurs sont reliées par la relation:

$$R_{ST} = C_{EB} \times S_{ST} \times \frac{1}{100}$$

**Tableau I**

| Catalyseur | A | B | C | D | E | F | G | H |
|---|---|---|---|---|---|---|---|---|
| Exemple | 1 | 2 | 3 | 4 | 5 | 6 | 7 | 8 |
| Convers. EB % mol. | 59,9 | 60,0 | 60,1 | 59,4 | 58,8 | 60,2 | 59,5 | 59,0 |
| Sélect. ST % mol. | 92,0 | 92,1 | 90,1 | 92,5 | 92,4 | 90,0 | 92,5 | 92,5 |
| Rendem. ST % mol. | 55,1 | 55,3 | 54,1 | 54,9 | 54,3 | 54,2 | 55,0 | 54,6 |

| Catalyseur | I | J | K | L | M | N | O |
|---|---|---|---|---|---|---|---|
| Exemple | 9 | 10 | 11 | 12 | 13 | 14 | 15 |
| Convers. EB % mol. | 61,0 | 60,0 | 60,5 | 60,1 | 65,9 | 61,0 | 60,5 |
| Sélect. ST % mol. | 90,5 | 92,3 | 90,1 | 90,5 | 91,9 | 92,0 | 90,4 |
| Rendem. ST % mol. | 55,2 | 55,4 | 54,5 | 54,4 | 60,5 | 56,1 | 54,7 |

Dans le tableau 1, la comparaison entre le catalyseur A et les catalyseurs C, D et E, la comparaison entre le catalyseur B et les catalyseurs F, G et H, la comparaison entre le catalyseur I et le catalyseur K, la comparaison entre le catalyseur J et le catalyseur L et la comparaison entre le catalyseur N et le catalyseur O montrent que la mise en jeu du lanthane apporte des améliorations dans le rendement en styrène par rapport à l'utilisation d'autres terres rares: cerium, praséodyme, néodyme; et cela que le métal de terre rare soit utilisé comme seul métal additionnel (en plus du fer, du chrome et du potassium), avec ou sans adjonction de kaolin, que le métal de terre rare soit associé à un autre métal additionnel sous la forme de deux composés métalliques séparés ou sous la forme d'une association d'oxydes préparés dans une étape préalable (cas des associations avec le cobalt et le vanadium).

0 112 754

**Revendications**

1. - Catalyseur renfermant sous forme d'oxydes du fer, du chrome et du potassium, le fer, le chrome et le potassium, comptés en oxydes, étant présents dans les rapports pondéraux:

$$\frac{FeO_3}{K_2O} \quad \text{de } 1/1 \text{ à } 10/1$$

$$\frac{CrO_3}{K_2O} \quad \text{de } 0,05/1 \text{ à } 0,4/1$$

$$\frac{Fe_2O_3}{CrO_3} \quad \text{de } 15/1 \text{ à } 40/1,$$

caractérisé en ce qu'il renferme également sous forme d'oxyde du lanthane en une proportion, comptée en oxyde, de 1 à 15 % en poids par rapport au poids du catalyseur.

2. - Catalyseur selon la revendication 1, caractérisé en ce qu'il renferme de la kaliophyllite de formule $Al_2O_3$, 2 $SiO_2$, $K_2O$ et du potassium en excès par rapport à celui présent dans la kaliophyllite, la proportion de kaliophyllite étant de 5 à 40 % du poids du catalyseur, l'excès de potassium étant avec le fer et le chrome dans les rapports pondéraux:

$$\frac{Fe_2O_3}{\text{excès de } K_2O} = 3/1 \text{ à } 10/1 \quad \text{et} \quad \frac{CrO_3}{\text{excès de } K_2O} = 0,1/1 \text{ à } 4/1.$$

3. - Catalyseur selon l'une des revendications 1 et 2, caractérisé en ce que les rapports pondéraux entre le fer, le chrome et le potassium, comptés en oxydes, sont:

$$\frac{Fe_2O_3}{K_2O \text{ total}} \quad \text{de } 2/1 \text{ à } 7/1 \; ;$$

$$\frac{CrO_3}{K_2O \text{ total}} \quad \text{de } 0,1/1 \text{ à } 0,3/1 \; ; \text{ et}$$

11

$$\frac{Fe_2O_3}{CrO_3} \quad \text{de } 25/1 \text{ à } 35/1,$$

et la proportion de lanthane, compté en oxyde, est de 3 à 10 % en poids.

4. - Catalyseur selon l'une des revendications 1 à 3, caractérisé en ce qu'il renferme également, compté en oxyde, de 0,1 à 5 % en poids d'au moins un métal choisi parmi le cobalt et le vanadium.

5. - Procédé de fabrication d'un catalyseur selon l'une des revendications 1 à 4, caractérisé en ce que:

a) on constitue un mélange comprenant de l'eau, au moins un composé du fer, au moins un composé du chrome, au moins un composé du potassium et au moins un composé de lanthane et on malaxe pour former une pâte et,

b) en sèche ladite pâte, telle quelle ou après mise en forme et on l'active thermiquement à une température de 850 à 1100°C,

les composés de fer, de chrome et de potassium étant engagés en des proportions correspondant en oxydes, aux rapports pondèraux suivants:

$$\frac{Fe_2O_3}{K_2O} \quad \text{de } 1/1 \text{ à } 10/1 \text{ ;}$$

$$\frac{CrO_3}{K_2O} \quad \text{de } 0,05\ /1 \text{ à } 0,4/1 \text{ ; et}$$

$$\frac{Fe_2O_3}{CrO_3} \quad \text{de } 15/1 \text{ à } 40/1.$$

et le composé de lanthane étant engagé en une proportion comptée en oxyde de 1 à 15 % en poids de l'ensemble des composés mis en jeu.

6. - Procédé selon la revendication 5, caractérisé en ce que l'on ajoute au moins un composé d'au moins un métal choisi parmi le cobalt et le vanadium au mélange des composés de métaux et du matériau argileux de l'étape (a), la teneur totale en les métaux ajoutés, exprimée en oxydes étant de 0,1 à 5 % en poids de l'ensemble des composés mis en jeu.

7. - Procédé selon l'une des revendications 5 et 6, caractérisé en ce que, dans l'étape (a), on met en jeu un matériau argileux en une proportion de 3,5 à 30 % en poids, le composé de potassium est mis en jeu en excès par rapport à la quantité capable de se combiner avec ledit matériau argileux, l'excès de composé de potassium étant avec le fer et le chrome en des proportions, comptées en oxydes, correspondant aux rapports pondéraux

$$\frac{Fe_2O_3}{\text{excès de } K_2O} \quad \text{de } 3/1 \text{ à } 10/1 \qquad \frac{CrO_3}{\text{excès de } K_2O} \quad \text{de } 0,1/1 \text{ à } 0,4/1.$$

8. - Procédé selon la revendication 7, caractérisé en ce que ledit matériau argileux est constitué au moins en partie de kaolinite.

9. - Procédé selon l'une des revendications 5 à 8, caractérisé en ce que la température d'activation de l'étape (b) est de 870 à 1050° C

10. - Utilisation d'un catalyseur selon l'une des revendications 1 à 4, dans une réaction de déshydrogénation d'un hydrocarbure aliphatique saturé ou monoéthylénique ou d'un hydrocarbure alkylaromatique.

11. - Utilisation selon la revendication 10, caractérisée en ce que ledit hydrocarbure alkylaromatique à déshydrogéner est l'éthylbenzène.

**Patentansprüche**

1. Katalysator, der in Form von Oxiden Eisen, Chrom und Kalium enthält, wobei Eisen, Chrom und Kalium, gerechnet als Oxide, in folgenden Gewichtsverhältnissen vorgesehen sind:

$$\frac{Fe_2O_3}{K_2O} \quad \text{von } 1/1 \text{ bis } 10/1$$

$$\frac{CrO_3}{K_2O} \quad \text{von } 0,05/1 \text{ bis } 0,4/1$$

$$\frac{Fe_2O_3}{CrO_3} \quad \text{von } 15/1 \text{ bis } 40/1,$$

dadurch gekennzeichnet, daß er ebenfalls in Form des Oxids Lanthan in einem Anteil, gerechnet als Oxid, von 1 bis 15 Gew.-%, bezogen auf das Gewicht des Katalysators, enthält.

2. Katalysator nach Anspruch 1, dadurch gekennzeichnet, daß er Kaliophyllit der Formel $Al_2O_3$, 2 $SiO_2$, $K_2O$ sowie Kalium im Überschuß zu dem bereits im Kaliophyllit vorhandenen enthält, wobei der Kaliophyllitanteil 5 bis 40 Gew.-% des Katalysators ausmacht und der Kaliumüberschuß mit dem Eisen und dem Chrom in folgenden Gewichtsverhältnissen vorliegt:

$$\frac{Fe_2O_3}{\text{Überschuß von } K_2O} = 3/1 \text{ bis } 10/1$$

und

$$\frac{CrO_3}{\text{Überschuß von } K_2O} = 0,1/1 \text{ bis } 0,4/1.$$

3. Katalysator nach einem der Ansprüche 1 und 2, dadurch gekennzeichnet, daß die Gewichtsverhältnisse zwischen Eisen, Chrom und Kalium, gerechnet als Oxide, betragen:

$$\frac{Fe_2O_3}{K_2O \text{ gesamt}} \quad \text{von } 2/1 \text{ bis } 7/1;$$

$$\frac{CrO_3}{K_2O \text{ gesamt}} \qquad \text{von } 0,1/1 \text{ bis } 0,3/1; \text{ und}$$

$$\frac{Fe_2O_3}{CrO_3} \qquad \text{von } 25/1 \text{ bis } 35/1,$$

und der Anteil an Lanthan, gerechnet als Oxid, 3 bis 10 Gew.-% beträgt.

4. Katalysator nach einem der Ansprüche 1 bis 3, dadurch gekennzeichnet, daß er darüberhinaus, gerechnet als Oxid, zwischen 0,1 bis 5 Gew.-% wenigstens eines Metalls enthält, daß aus Kobalt und Vanadium gewählt ist.

5. Verfahren zur Herstellung eines Katalysators nach einem der Ansprüche 1 bis 4, dadurch <u>gekennzeichnet,</u> daß:

a) man ein Gemisch bildet, das enthält: Wasser, wenigstens eine Verbindung des Eisens, wenigstens eine Verbindung des Chroms, wenigstens eine Verbindung des Kaliums und wenigstens eine Lanthanverbindung und daß man zur Bildung einer Paste knetmischt und

b) daß man diese Paste so, wie sie ist oder nach Formgebung trocknet und thermisch bei einer Temperatur von 850 bis 1100°C aktiviert, wobei die Verbindungen des Eisens, Chroms und Kaliums in Anteilen eingebunden sind, die als Oxide folgenden Gewichtsverhältnissen entsprechen:

$$\frac{Fe_2O_3}{K_2O} \qquad \text{von } 1/1 \text{ bis } 10/1;$$

$$\frac{CrO_3}{K_2O} \qquad \text{von } 0,05/1 \text{ bis } 0,4/1; \text{ und}$$

$$\frac{Fe_2O_3}{CrO_3} \qquad \text{von } 15/1 \text{ bis } 40/1,$$

und die Lanthanverbindung in einem Anteil, gerechnet als Oxid, zwischen 1 bis 15 Gew.-% der Gesamtheit der in Frage kommenden Verbindungen eingebunden ist.

6. Verfahren nach Anspruch 5, dadurch gekennzeichnet, daß man wenigstens eine Verbindung wenigstens eines Metalls, gewählt aus Kobalt und Vanadium, dem Gemisch der Metallverbindungen der Stufe (a) zusetzt, wobei der Gesamtgehalt an diesen zugesetzten Metallen, ausgedrückt als Oxide, zwischen 0,1 bis 5 Gew.-% der Gesamtheit der eingesetzten Verbindungen beträgt

7. Verfahren nach einem der Ansprüche 5 und 6, dadurch gekennzeichnet, daß in der Stufe (a) ein tonhaltiges Material in einem Anteil von 3,5 bis 30 Gew.-%, die Kaliumverbindung im Überschuß, bezogen auf die Menge genommen wird, die in der Lage ist, sich mit diesem tonhaltigen Material zu kombinieren, wobei der Überschuß der Kaliumverbindung mit dem Eisen und dem Chrom in Anteilen, gerechnet als Oxide, entsprechend den Gewichtsverhältnissen

$$\frac{Fe_2O_3}{\text{Überschuß von } K_2O} \qquad \text{von } 3/1 \text{ bis } 10/1$$

$$\frac{CrO_3}{\text{Überschuß von } K_2O} \qquad \text{von } 0,1/1 \text{ bis } 0,4/1$$

beträgt.

8. Verfahren nach Anspruch 7, dadurch gekennzeichnet, daß dieses tonhaltige Material wenigstens zum Teil aus Kaolinit besteht.

9. Verfahren nach einem der Ansprüche 5 bis 8, dadurch gekennzeichnet, daß die Aktivierungstemperatur der Stufe (b) 870 bis 1050°C beträgt.

10. Verwendung eines Katalysators nach einem der Ansprüche 1 bis 4 in einer Dehydrogenisierungsreaktion eines gesättigten aliphatischen oder monoethylenischen Kohlenwasserstoffs oder eines alkylaromatischen Kohlenwasserstoffs.

11. Verwendung nach Anspruch 10, dadurch gekennzeichnet, daß dieser zu dehydrogenisierende alkylaromatische Kohlenwasserstoff Äthylbenzol ist.

## Claims

1.- A catalyst containing as oxides iron, chromium and potassium, the iron, chromium and potassium calculated as oxides, being present in the weight ratios:

$$\frac{Fe_2O_3}{K_2O} \qquad \text{from } 1/1 \text{ to } 10/1$$

$$\frac{CrO_3}{K_2O} \qquad \text{from } 0.05/1 \text{ to } 0.4/1$$

$$\frac{Fe_2O_3}{CrO_3} \qquad \text{from } 15/1 \text{ to } 40/1,$$

characterized in that it also contains, as oxide, lanthanum in a proportion, calculated as oxide, from 1 to 15% by weight with respect to the catalyst weight.

2.- A catalyst according to claim 1, characterized in that it contains kaliophyllite of the formula $Al_2O_3$, $2SiO_2$, $K_2O$ and an excess of potassium with respect to that comprised in kaliophyllite, the kaliophyllite proportion being from 5 to 40% of the catalyst weight, the potassium excess being, with respect to iron and chromium, in the weight ratios:

$$\frac{Fe_2O_3}{K_2O \text{ excess}} = 3/1 \text{ to } 10/1 \text{ and } \frac{CrO_3}{K_2O \text{ excess}} = 0.1/1 \text{ to } 4/1.$$

3.- A catalyst according to one of claims 1 and 2, characterized in that the weight ratios between iron, chromium and potassium, calculated as oxides, are:

$$\frac{Fe_2O_3}{Total\ K_2O} \qquad from\ 2/1\ to\ 7/1;$$

$$\frac{CrO_3}{Total\ K_2O} \qquad from\ 0.1/1\ to\ 0.3/1;\ and$$

$$\frac{Fe_2O_3}{CrO_3} \qquad from\ 25/1\ to\ 35/1,$$

and the lanthanum proportion, calculated as oxide, is from 3 to 10% by weight.

4.- A catalyst according to one of claim 1 to 3, characterized in that it also contains, calculated as oxide, from 0.1 to 5% by weight of at least one metal selected from cobalt and vanadium.

5.- A process for manufacturing a catalyst according 60 to one of claims 1 to 4, characterized in that:

a) a mixture is formed which comprises water, at least one iron compound, at least one chromium compound, at least one potassium compound and at least one lanthanum compound, and is malaxed to form a paste, and

b) said paste is dried, as such or after shaping, and is thermally activated at a temperature from 850° to 1100°C, the iron, chromium and potassium compounds being used in proportions corresponding, as oxides, to the following weight ratios:

$$\frac{Fe_2O_3}{K_2O} \qquad from\ 1/1\ to\ 10/1;$$

$$\frac{CrO_3}{K_2O} \qquad from\ 0.05/1\ to\ 0.4/1;\ and$$

$$\frac{Fe_2O_3}{CrO_3} \qquad from\ 15/1\ to\ 40/1,$$

and the lanthanum compound being used in a proportion, calculated as oxide, from 1 to 15% by weight of the totality of the compounds involved.

6.- A process according to claim 5, characterized in that at least one compound of at least one metal selected from cobalt and vanadium is added to the metal compounds of step (a), the total content of added metals, expressed as oxides, being from 0.1 to 5% by weight of the totality of the compounds involved.

7.- A process according to one of claims 5 and 6, characterized in that, in step (a), a clayish material is used in a proportion from 3.5 to 30% by weight, the potassium compound is used in excess with respect to the amount liable to combine with said clayish material, the potassium compound excess being with respect to iron and chromium in proportions, calculated as oxides, corresponding to the weight ratios:

$$\frac{Fe_2O_3}{K_2O\ excess}\ from\ 3/1\ to\ 10/1 \qquad \frac{CrO_3}{K_2O\ excess}\ from\ 0.1/1\ to\ 0.4/1$$

8.- A process according to claim 7, characterized in that said clayish material is comprised at least partly of kaolinite.

9.- A process according to one of claims 5 to 8, characterized in that the activation temperature of step (b) is from 870° to 1050°C.

10.- The use of a catalyst according to one of claims 1 to 4, in a dehydrogenation reaction of a saturated or monoethylenic aliphatic hydrocarbon or an alkylaromatic hydrocarbon.

11.- The use according to claim 10, characterized in that said alkylaromatic hydrocarbon to be dehydrogenated is ethylbenzene.